# FASCICULE DE BREVET EUROPEEN

(11) **EP 2 288 342 B1**
(45) Date de publication et mention de la délivrance du brevet: **28.06.2017**
(21) Numéro de dépôt: 09761875.5
(22) Date de dépôt: 20.05.2009
(51) Int. Cl.: A61K 9/20, A61K 9/28, A61K 33/06, A61K 9/06, A61K 9/70

(54) **SYSTEME A BASE DE MAGNESIUM ET SON UTILISATION EN COSMETIQUE**
MAGNESIUM-SYSTEM UND SEINE VERWENDUNG IN DER KOSMETISCHEN INDUSTRIE
MAGNESIUM SYSTEM AND USE THEREOF IN THE COSMETICS INDUSTRY

(30) Priorité: 20.05.2008 FR 0802702; 30.07.2008 FR 0804349
(43) Date de publication de la demande: 02.03.2011
(73) Titulaire: Joanny, Fabienne, 75008 Paris (FR)
(72) Inventeur: Joanny, Fabienne, 75008 Paris (FR)
(74) Mandataire: Tezier Herman, Béatrice
(86) Numéro de dépôt international: PCT/FR2009/000586
(87) Numéro de publication internationale: WO 2009/150324

(56) Documents cités:
- WO-A-2005/049053
- WO-A-2005/091872
- US-A- 5 898 037
- US-A1- 2004 156 896
- US-A1- 2005 220 865
- US-A1- 2005 266 082
- US-A1- 2006 217 385

## Description

### Domaine de l'invention

La présente invention a trait à un nouveau système à base de magnésium et à son utilisation en cosmétique pour le soin et la santé de la peau et des phanères, notamment vis-à-vis du stress de la peau, ledit système comprenant une préparation orale contenant une source de magnésium, d'une part, et une préparation topique contenant également une source de magnésium, d'autre part.

### Art antérieur

On connait des demandes de brevet US 2005/220865, US 2006/217385, US 2005/266082 et US 2004/156896 des compositions pharmaceutiques orales, en général des comprimés, qui comprennent les substances suivantes : magnésium ou dérivé de magnésium, hydroxypropylcellulose, lactose, silice colloïdale et béhénate de glycéryle choisi dans une liste d'agents possibles. Aucun de ces document ne prévoient toutefois des compositions à administration orale comprenant l'ensemble de ces agents aux quantité s spécifiées selon 1 ?invention et une combinaison avec une forme topique. Dans l'organisme, le magnésium joue sous la forme ionique Mg²⁺ un rôle important dans de nombreuses réactions biochimiques. Il agit comme un cofacteur essentiel dans toutes les réactions du métabolisme énergétique impliquant l'adénosine triphosphate (ATP). De plus, il est nécessaire à l'interaction entre actine et myosine, à la base de la contraction musculaire. Il est également nécessaire à la synthèse des fibres musculaires. Chez l'homme, le magnésium est, après le calcium et le phosphore, le minéral le plus abondant dans l'organisme. Moins de 1% du magnésium total se trouve dans le sérum, environ 60%, se trouve dans la trame minérale de l'os, 30 % se trouve intracellulairement dans les muscles, le reste se trouve intracellulairement dans les autres tissus mous.

Au niveau cellulaire, le magnésium participe aux échanges ioniques transmembranaires des cations. On sait en particulier des articles de DENDA, M., Adv. Drug Deliv. Rev., 2002 Nov., 1;54 Suppl. 1:S123-130 et DENDA M. et al., J. Invest. Dermatol., 2002 Jan., 118(1):65-72, que le magnésium agit en association étroite avec le sodium, le potassium et le calcium, avec lesquels il doit rester en équilibre dans l'organisme : en effet, pour être en bonne santé, la cellule doit pouvoir exercer facilement des changements de charge ionique ; les cations Ca²⁺ et le Mg²⁺ dépolarisent la cellule en y entrant ; les cations Na⁺ et K⁺ polarisent la cellule en en sortant et rétablissent ainsi le retour de la cellule à son état initial.

Le magnésium est un cofacteur indispensable au fonctionnement des différentes pompes membranaires (Ca²⁺ et Mg²⁺)-dépendantes. Un déficit en magnésium intracellulaire est susceptible de contribuer au blocage des différentes pompes réticulaires et donc d'inhiber la sortie du calcium. Ainsi, au niveau des fibres musculaires, l'augmentation du calcium libre intramyoplasmique est responsable de contractures spontanées, d'épuisement des réserves en ATP et à terme de lésions tissulaires.

On sait en particulier que le magnésium administré par voie orale, eu égard à ses effets polyvalents, notamment sur les symptômes associés aux syndromes de surmenage et de fatigue, est largement utilisé, seul ou en association, afin de répondre à divers états de stress :
- stress lié à la fatigue, au surmenage, au sport intensif,
- troubles du sommeil, insomnies, anxiété, et
- arrêt du tabac, sevrage alcoolique.

En outre, des études cliniques ont démontré l'efficacité des sels de magnésium dans le traitement ou la prévention de différentes situations pathologiques :
- symptômes associés à la ménopause ou au syndrome prémenstruel (efficacité améliorée en association avec la vitamine B6),
- prévention de l'infarctus du myocarde, traitement d'appoint des arythmies et de l'hypertension, et
- autres domaines (prévention du diabète et de l'ostéoporose, soutien de l'effort musculaire, amélioration des performances physiques).

Ces situations pathologiques se rencontrent très souvent dans les cas de déficience en magnésium.

En outre, on sait, notamment des documents de brevet EP 0542979 B et WO 2004/105778 A, que le magnésium administré par voie orale est utile en cosmétique notamment pour lutter contre les manifestations du stress de la peau et celles de la fatigue de la peau.

De la publication WO 2005/049053 A, on a proposé pour lutter contre le dysfonctionnement sexuel l'utilisation de comprimés, gélules, préparations injectables ou préparations topiques contenant du, magnésium. Ce document ne décrit ni ne suggère la combinaison d'un comprimé avec une préparation d'usage topique.

Le brevet délivré US 5898037 A préconise pour le traitement de l'acné, de l'arthrite, des maladies du parodonte, des maladies ophtalmiques telles que la conjonctivite, des hémorroïdes, des infections et inflammations vaginales, des préparations à base de magnésium. Ce brevet mentionne (i) l'administration d'une préparation topique contenant du Mg et (ii) pour compléter l'effet de celle-ci, l'administration d'une préparation orale contenant également du Mg (voir colonne 3, lignes 11-13 et 30-32, d'une part, et colonne 8 lignes 58-67, d'autre part). L'inconvénient de la préparation orale complémentaire réside dans le fait qu'elle doit fournir 300 à 900 mg/j de magnésium, une quantité énorme eu égard à l'article de ROTH P. et al., 'Intestinal Absorption of Magnesium in Man', International Journal of Applied Radiation and Isotopes, 1979:30, 523-526, signalant que, par administration orale de l'isotope ²⁸Mg chez l'homme, la biodisponibilité, exprimée sous la forme de pourcentage de magnésium absorbé par rapport à la quantité pondérale de magnésium administré, décroît quand la dose de magnésium augmente. En outre ledit brevet ne décrit ni ne suggère la caractéristique de la présente invention, à savoir que la préparation orale est à libération prolongée et continue.

On sait également que le magnésium, administré par voie topique, agit également sur le stress et la fatigue de la peau, d'une part, et la régénération de la barrière cutanée, ce qui améliore l'hydratation de la peau, d'autre part.

Or on vient de constater, d'une part, que l'administration orale de magnésium ne permet pas un apport suffisant en ions Mg²⁺ au niveau des couches les plus externes de l'épiderme telles que la couche cornée **(*stratum corneum*)** et, d'autre part, que l'administration de magnésium par voie topique ne permet pas un apport suffisant en ions Mg²⁺ au niveau des couches internes de la peau, notamment l'hypoderme et la partie interne du derme, quand le ***stratum corneum*** est carencé en Mg. Il s'agit là d'un déséquilibre biologique auquel la présente invention se propose de remédier.

### But de l'invention

Selon l'invention on se propose de fournir une nouvelle solution technique pour résoudre le problème, qui se pose, de l'irrigation de la peau en ions Mg²⁺ dans toute son épaisseur, d'une part, et pour favoriser l'assimilation par l'ensemble de la peau du magnésium d'origine topique, qui est en général stoppé par la couche cornée (on veut ici, selon l'expression de la personne du métier : "booster" le Mg topique), d'autre part.

### Objet de l'invention

Ce but a été atteint par le biais de la combinaison, non suggérée ni décrite dans l'art antérieur, d'une administration d'une préparation orale, sous forme de comprimé, contenant une source de magnésium avec une administration d'une préparation topique contenant du magnésium, en vue d'avoir au niveau de la peau (i) la dualité des effets 'interne/externe' (ou 'inside/outside') des préparations orale et topique.

Selon un premier aspect de l'invention, on préconise un système à base de magnésium, utilisable pour les soins de la peau, qui est caractérisé en ce qu'il comprend :
(a) une première source de magnésium dans une préparation orale, sous forme de comprimé à libération progressive, la dite source fournissant des ions Mg²⁺ dans l'organisme, la forme comprimé présentant *in vitro,* après 2h en milieu HCl 0,1N, un taux de dissolution (δ) du magnésium, qu'elle contient, inférieur ou égal à 60 %, en poids par rapport au poids total du Mg fourni par ladite source, et
(b) une seconde source de magnésium dans une préparation topique, ladite source fournissant des ions Mg²⁺ au niveau de la peau.

Selon un second aspect de l'invention, on préconise une trousse de soin, ladite trousse étant caractérisée en ce qu'elle contient ladite préparation orale et ladite préparation topique dudit système.

Selon un troisième aspect de l'invention, on préconise une nouvelle utilisation du système de l'invention pour le soin de la peau vis-à-vis du stress, de la fatigue et des déficiences de la barrière cutanée notamment en ce qui concerne l'hydratation.

En bref le système selon l'invention intervient en tant que cosmétique. Il est plus particulièrement destiné (a) pour l'hydratation de la peau, et (b) pour traiter ou prévenir le stress de la peau.

### Description détaillée de l'invention

### A. Les sources de magnésium

Les sources de magnésium utiles selon l'invention, qui sont identiques ou différentes, sont chacune un dérivé physiologiquement acceptable de magnésium. En ce qui concerne la préparation topique, ledit dérivé physiologiquement acceptable de magnésium est choisi parmi l'ensemble constitué par
(α) l'oxyde de magnésium, MgO,
(β) les sels de Mg avec des acides inorganiques,
(γ) les sels de Mg avec des acides organiques,
(δ) les hydrates desdits sels inorganique et organiques, et
(ε) leurs mélanges.

Parmi les acides inorganiques utilisables pour l'obtention des sels (β), on peut citer HCl et H₂SO₄. Parmi les acides organiques utilisables pour l'obtention des sels (γ), on peut signaler :
- les acides simples, tels que notamment l'acide acétique, l'acide propionique, et l'acide isobutyrique (ou acide 2-méthyl-2-propionique),
- les polyacides tels que notamment l'acide oxalique, l'acide maléique, l'acide fumarique, l'acide malonique, l'acide citraconique (ou acide 2-méthyl-2-butènedioïque),
- les hydroxyacides, tels que notamment l'acide malique, l'acide citrique, l'acide tartrique, l'acide lactique, l'acide salicylique, l'acide vanillique, l'acide gluconique, l'acide glucuronique, l'acide glycérophosphorique, l'acide mandélique et l'acide citramalique (ou acide 2-hydroxy-2-méthylbutanedioïque)
- les aminoacides naturels ou non naturels, tels que notamment l'acide aspartique, l'acide glutamique, l'asparagine, la lysine, l'acide pidolique (autres nomenclatures : acide pyroglutamique ou 5-oxo-L-proline), l'acide pyridine-2-carboxylique, l'acide pyridine-3-carboxylique, l'acide pyridine-4-carboxylique, l'acide aminobutanedioïque et l'acide orotique.

Les hydrates (δ) comprennent notamment les hydrates de MgCl₂ de formule (I) :

MgCl₂.n(H₂O) (I)

dans laquelle n est un nombre entier ou fractionnaire ayant pour valeur 1 à 6 (de préférence 1 à 9/2).

Parmi les mélanges (ε), qui conviennent, on peut mentionner le magnésium marin. Il s'agit d'un mélange d'origine marine contenant au moins 70 % en poids de sels inorganiques de Mg. La principale production de magnésium marin provient de l'exploitation de la Mer Morte.

Pour les formes topiques, on recommande avantageusement de faire appel aux sources (β)-(ε). D'une manière générale, pour lesdites formes topiques, on préconise plus particulièrement MgCl₂, les hydrates MgCl₂.n(H₂O) où n est un nombre entier ou fractionnaire ayant pour valeur 1 à 9/2, le magnésium marin, ou un sel de Mg avec l'acide aspartique, l'acide glutamique, l'asparagine, la lysine, l'acide pidolique ou l'acide orotique.

En ce qui concerne la préparation orale, ledit dérivé physiologiquement acceptable de magnésium est choisi parmi l'ensemble constitué par MgO, MgCl₂ et les hydrates de formule MgCl₂.n(H₂O) où n est un nombre entier ou fractionnaire supérieur à 0 et inférieur ou égal à 6. Les sels de Mg avec les acides organiques ne conviennent généralement pas ici (notamment quand il s'agit de sels d'acides gras). En effet, (a) le pourcentage pondéral du magnésium dans ces sels diminue lorsque la masse moléculaire augmente, et (b) par suite ces sels conduisent à des comprimés de taille et masse trop importantes qu'il devient difficile de les avaler. En effet, quand la masse moléculaire de la source de magnésium du comprimé augmente, la teneur en Mg fourni par ladite source diminue. Si la teneur en Mg dans MgO est de 60 % en poids, elle est de 25,5 % en poids dans MgCl₂, et de 13,7 % en poids dans MgCl₂.9/2(H₂O). En conséquence, quand on utilise un comprimé, il est plutôt intéressant d'utiliser une source minérale de Mg telle que MgCl₂, MgCl₂.n(H₂O) où n a pour valeur 1 à 9/2, ou le magnésium marin, pour limiter les dimensions dudit comprimé. La source préférée selon l'invention est un hydrate, à savoir MgCl₂.9/2H₂O.

### B. Les préparations orales

Les préparations orales selon l'invention sont, comme indiqué plus haut, des comprimés à libération progressive. Leur source de Mg apporte une quantité représentant environ 90 à 110 parties en poids de Mg.

Les préparations avantageuses selon l'invention sont des comprimés qui présentent *in vitro,* après 2h en milieu HCl 0,1N, un taux de dissolution (δ) du magnésium dissous compris entre 20 % et 60 % en poids par rapport au poids du magnésium apporté par la source de magnésium (i. e. 20 % ≤ δ ≤ 60 %) et de préférence un taux de dissolution compris entre 25 % et 58 % (i. e. 25 % ≤ δ ≤ 58 %).

Ces préparations orales comprennent :
(I) les comprimés à une couche (dits 'comprimés monocouches') contenant la totalité de la source de magnésium, et
(II) les comprimés à deux couches (dits 'comprimés bicouches') comprenant
   (a) une première couche (ou couche ou noyau 'interne') gastrorésistante, ou logée dans une enveloppe gastrorésistante, ladite première couche contenant 80 à 40 % du magnésium apporté par la source de magnésium, et
   (b) une seconde couche (ou couche 'externe') qui est hydrophile, se dissout au niveau de l'estomac et contient 20 à 60 % du magnésium apporté par la source de magnésium.

### C. Préparation orale particulièrement préférée

Selon l'invention, on recommande tout particulièrement une préparation orale du type monocouche qui est un comprimé à libération progressive, qui comprend une matrice contenant une source de magnésium, ladite matrice étant dépourvue d'un enrobage gastrorésistant mais pourvue d'un enrobage de protection ralentissant ou freinant la dissolution du Mg au niveau gastrique. Une telle préparation orale est décrite dans la demande internationale parente, déposée le même jour que la présente demande et intitulée : *'Utilisation d'une matrice pour administration orale de magnésium à libération prolongée, et composition contenant cette matrice'*

Dans ce cas particulier, le système selon l'invention comporte en tant que préparation orale un comprimé, ledit comprimé se présentant sous la forme d'une matrice enrobée pour l'administration orale de magnésium avec libération progressive, ladite matrice étant dépourvue d'un enrobage gastrorésistant mais pourvue d'un enrobage de protection ralentissant ou freinant la dissolution du Mg au niveau gastrique, ladite matrice, qui est constituée de ladite source de magnésium (A), d'un agent de retard hydrophile (B1), d'un agent de retard hydrophobe (B2), d'une charge inerte (C1) intervenant en tant que diluant et d'une charge inerte (C2) intervenant en tant que moyen lubrifiant, comprenant, pour administrer
(A) 90 à 110 parties en poids de magnésium provenant d'une source choisie parmi MgO, MgCl₂, les hydrates de formule MgCl₂.n(H₂O), où n est un nombre entier ou fractionnaire ayant pour valeur 1 à 9/2, et leurs mélanges, les ingrédients suivants :
   (B1) 180 à 190 parties en poids d'hydroxypropylméthylcellulose,
   (B2) 19,8 à 22,2 parties en poids de béhénate de glycéryle,
   (C1) 10 à 12 parties en poids de lactose, et
   (C2) 10 à 12 parties en poids de silice colloïdale.

Avantageusement ledit enrobage comprend
(D) 15 à 75 parties et de préférence 15 à 45 parties en poids d'une substance choisie parmi la gomme laque, les éthers de cellulose (notamment HPMC et HPC) et leurs mélanges.

Selon l'invention, le rapport pondéral B1/B2 est compris entre 180/22,2 = 8,1/1 et 190/19.8 = 9,6/1. Avantageusement, on recommande que ledit rapport pondéral soit compris entre 8,5/1 et 9,3/1. De préférence, le rapport pondéral B1/B2 se situera entre 8,7/1 et 9,2/1, par exemple : 8,8/1 ou 9/1 ou encore 9,15/1.

Le lactose, composant C1, est avantageusement anhydre. De même la silice colloïdale, composant C2, est avantageusement anhydre. En pratique, il est plutôt préféré que dans la matrice de l'invention le rapport pondéral C1/C2 soit voisin de 1/1 et mieux égal à 1/1.

L'enrobage de la matrice n'est pas gastrorésistant. Il s'agit d'un pelliculage qui intervient (i) pour protéger les composants de la matrice vis-à-vis de l'extérieur, notamment vis-à-vis des chocs, et surtout (ii) pour ralentir la dissolution du Mg dans la phase 'gastrique'. Ce pelliculage peut être réalisé en une seule couche, deux couches, voire même trois couches. Pour limiter les coûts de fabrication, il est possible qu'il soit monocouche. Toutefois, l'on recommande avantageusement un enrobage à deux couches pour mieux contrôler la dissolution du Mg. L'enrobage de la matrice représente en général 15 à 75 parties en poids (i. e. approximativement 1,3 à 7,5 % en poids par rapport au poids de la matrice), et de préférence 15 à 70 parties en poids, et mieux 15 à 45 parties en poids, pour 90 à 110 parties en poids de Mg.

Les substances recommandées ici pour l'enrobage sont la gomme laque, et les éthers de cellulose filmogènes tels que les alkylcelluloses, à savoir plus particulièrement les mélanges de HPMC et d'hydroxypropylcellulose (HPC) commercialisés notamment sous les nomenclatures de NUTRATERIC^{®} et OPADRY^{®}. On peut également envisager un enrobage constitué d'une première couche de gomme laque et d'une couche externe faite d'un mélange d'alkylcelluloses.

En pratique, on préconise un enrobage qui est
(a) un pelliculage monocouche de gomme laque (utilisée à 50 % en poids dans de l'éthanol, le solvant étant éliminé lors du pelliculage), ou
(b) un pelliculage à deux couches, chaque couche comprenant une substance choisie parmi la gomme laque, les éthers de cellulose (notamment HPMC et HPC) et leurs mélanges.

Quand on utilise un enrobage à deux couches, la première couche (ou couche interne) représente en général 0.5 à 4 % en poids par rapport au poids de la matrice, et la seconde couche (ou couche externe) représente en général 0,5 à 3.5 % en poids par rapport au poids de ladite matrice, l'ensemble des deux dites couches représentant 1,3 à 7,5 % en poids par rapport au poids de ladite matrice.

La cinétique de dissolution dudit comprimé, déterminée au moyen d'un système de dissolution comprenant d'abord le traitement du comprimé constituant la préparation orale dans un milieu [en particulier 90.0 ml, à 40 °C selon la méthode préconisée dans la pharmacopée américaine] HCl 0,1N de T = 0 à T = 2h, puis le traitement dans un tampon [en particulier 900 ml, à 40 °C] à pH 6,8 de T = 2h à T = 8h, a un taux de dissolution (δ) de Mg par rapport au Mg administré tel que
- à T = 2h, on a : δ ≤, 60 %, plus précisément : 20 % ≤ δ ≤ 60 %, et de préférence : 25 % ≤ δ ≤ 58 % ;
- à T = 4h, on a : δ ≤ 85 %, plus précisément : 40 % ≤ δ ≤ 85 %, et de préférence : 45 % ≤ δ ≤ 82 % ;
- à T = 6h, on a : δ ≤ 98 %, plus précisément : 60 % ≤ δ ≤ 98 %, et de préférence : 80 % ≤ δ ≤ 95 % ; et
- à T = 8h, on a : δ ≤ 100 %, plus précisément : 90 % ≤ δ ≤ 100 %, et de préférence : 95 % ≤ δ ≤ 99,9 %.

Dans cette technique d'évaluation de la cinétique de dissolution, les teneurs en Mg libéré sont déterminées par complexométrie à l'EDTA. La cinétique de dissolution peut être déterminée à une température de 15 à 40 °C, notamment à la température ambiante (15-25 °C). Cependant, comme les composants de la préparation orale et les comprimés qu'ils constituent sont des produits qui ne se dégradent pas lors du stockage pendant plusieurs mois à 40 °C, on a retenu ladite température de 40 °C pour apprécier ladite cinétique afin de se placer dans des conditions de température proches de celle de l'intérieur du corps humain.

### D. Les préparations topiques

Les préparations topiques utilisables selon l'invention comprennent notamment les crèmes, les gels, les onguents, les laits, les solutions aqueuses, les solutions hydro-alcooliques et les compositions pour nébulisation. Par exemple on peut utiliser une préparation topique choisie notamment parmi les :
- gel translucide hydratant,
- fluide démaquillant,
- gel douche relaxant,
- shampoing d'usage fréquent à pH neutre,
- lait apaisant,
- gel-crème apaisant,
- mousse purifiante,
- crème régénérante, et
- lait anti-âge.

En pratique, la source de magnésium d'une telle préparation topique contiendra avantageusement une teneur de 0,5 à 4 % en poids de Mg et de préférence 0,8 à 2 % en poids de Mg par rapport au poids total de ladite préparation topique.

### E. Essais

Le système selon l'invention est particulièrement utile en tant que cosmétique pour (i) l'hydratation de la peau, et/ou (ii) traiter ou prévenir le stress de la peau. L'intérêt cosmétique du Mg apporté par ce système au niveau de la peau peut être apprécié par :
- la mesure de l'impédance électrique de la peau (exprimée en Ω) ou son inverse, la conductance (exprimée en S), le taux d'hydratation de la peau étant inversement proportionnel à l'impédance et proportionnel à la conductance, voir à cet effet les méthodes décrites par KALIA Y. et al., Biophys. J 1996;71(5):2692-2700*,* KALIA Y. et al., J. Pharm. Sci. 1998;87(12):1508-1811*,* CURDY C. et al., AAPS Pharm. Sci. 2000;2(3):E23*,* et CLAR E. J. et al., J. Cosm. Chem. 1975;26:337-357 *;* et/ou
- l'analyse d'explants de peau humaine maintenus en survie.

### F. Posologie

En ce qui concerne la posologie, l'on recommande pour les comprimés précités la prise quotidienne d'un comprimé dosé à 50 ou 100 mg de magnésium (le matin), ou mieux la prise quotidienne de deux comprimés dosés chacun à 50 mg de magnésium (l'un le matin, l'autre le soir).

Pour les préparations topiques, l'on recommande (i) l'application par massage d'un gel, une à deux fois par jour, à raison de 0,2 à 0,8 cm³ de gel pour une surface de peau de 2 cm² par application, ou l'application d'un spray contenant 0,8 à 2 % en poids de Mg par rapport au poids total de ladite préparation topique, deux fois par jour.

### G. Trousse de soin

Il est avantageux, pour satisfaire l'utilisateur et assurer la régularité du traitement, de présenter sous un même conditionnement, une trousse, nécessaire, kit ou coffret de soin contenant une préparation orale et une préparation topique conformes au système selon l'invention.

### H. Conclusions

Le système selon l'invention agit sur la peau et les phanères, notamment au niveau des cheveux, selon un aspect dual. Plus précisément il exerce sur la peau
(1°) une action par voie externe (i) favorable sur la myorelaxation et le renforcement de la barrière cutanée (ce renforcement se traduisant par une augmentation de l'hydratation de la peau), et (ii) apaisante sur la peau ; et
(2°) une action par voie interne favorable sur la myorelaxation, utile contre les manifestations du stress, réparatrice, anti-inflammatoire et antioxydante (notamment vis-à-vis des radicaux libres d'origine extérieure ou générés par l'organisme).

En outre, le système selon l'invention a un effet équilibrant (équilibre ionique) dans l'épaisseur de la peau, par voie interne et externe.

Selon l'invention, la préparation orale, qui est avantageusement sous la forme comprimé à libération progressive, "booste" la préparation topique.

### I. Exemples

D'autres avantages et caractéristiques de l'invention seront mieux compris à la lecture qui va suivre d'exemples de réalisation. Bien entendu l'ensemble de ces exemples n'est pas limitatif mais est donné à titre d'illustration.

### Exemple 1

### (a) Comprimés [correspondant à l'Exemple 5 de la demande parente]

On a préparé des comprimés monocouches à libération progressive, dosés à 100 mg de magnésium chacun et ayant la formulation suivante (le rapport pondéral B1/B2 étant de 9,15/1), où l'abréviation "Qté/cp" désigne la quantité par comprimé.

| Constituants | Qté/cp (mg) |
|---|---|
| *Noyau* : | |
| MgCl2.9/2H2O | 725,0 |
| HPMC | 183,0 |
| Mono-diglycéride béhénate | 20,0 |
| Lactose anhydre | 11,0 |
| Silice colloïdale anhydre | 11,0 |

| *Pelliculage* : | |
|---|---|
| 1ère couche : Gomme laque | 19,8 |
| 2ème couche (externe) : Mélange HPMC/HPC 1/4 p/p | 19,8 |
| *Total* : | 989,60 |

### (b) Gel

On prépare un gel à partir d'une composition aqueuse contenant de la silice colloïdale hydrophile et 4 % en poids de pidolate de magnésium. Ce gel offre l'avantage de ne pas comporter des substances pouvant interférer dans le cadre des essais comparatifs (tels que polyols intervenant en tant que promoteurs de perméation, vitamine E, écran solaire, etc).

### (c) Conditionnement et posologie

On conditionne la préparation orale (a), sous forme de comprimé retard, et la préparation topique (b) dans une même trousse, la posologie étant de 1 comprimé par jour (prise effectuée le matin) et deux applications du gel par jour (une le matin et l'autre le soir).

### Exemple 2

On procède comme indiqué à l'exemple 1 ci-dessus, avec la différence (1°) que l'on prépare à l'étape (a) des comprimés monocouches à libération progressive dosés chacun à 50 mg de magnésium, et (2°) que à l'étape (c) on administre deux de ces comprimés dosés à 50 mg par jour (un le matin, l'autre le soir).

### Exemples 3 et 4

### (a) Comprimés

On procède comme indiqué à l'exemple 1(a) pour préparer des comprimés monocouches à libération progressive dosés chacun à 100 mg de magnésium et, respectivement, à l'exemple 2(a) pour préparer des comprimés à libération progressive dosés chacun à 50 mg de magnésium.

### (b) Spray

Comme préparation topique, on fait appel à un spray raffermissant ayant la formulation suivante :

| Composant | % (p/p) |
|---|---|
| Propylèneglycol | 2,30 |
| Edétate tétrasodique | 0,20 |
| Extrait de pomme | 0,25 |
| Extrait de jujube (fruit du jujubier) | 0,15 |
| Extrait de jujubier polyéthoxylé | 0,20 |
| Glycérides de noix de macadamia polyéthoxylés | 0,08 |
| Huile de palme | 0,03 |
| Polyacrylate de sodium | 0,02 |
| Vitamine E et conservateur | 0,06 |
| Fragrance | 1,00 |
| Orotate de magnésium | 1,75 |
| Eau déminéralisée | qsp 100 |

### (c) Conditionnement et posologie

Les conditionnement et posologie sont ceux indiqués aux exemples 1 (c) et, respectivement, 2(c).

### Exemples 5 et 6

On procède comme indiqué ci-dessus aux exemples 3 et, respectivement, 4 en utilisant, comme composition topique, une crème fluide ayant la formulation suivante :

| Composant | % (p/p) |
|---|---|
| Propylèneglycol | 2,00 |
| Hyaluronate de sodium | 5,00 |
| Cyclométhicone | 5,00 |
| Mélange diméthicone/trisiloxane/ceteth-10/laureth-4 | 3,80 |
| Mélange cyclométhicone/diméthiconol | 3,50 |
| Huile d'argan | 2,50 |
| 3-Hydroxy-L-proline (20 % p/v dans H₂O) | 2,00 |
| Extrait d'avoine (oat kernel extract) | 1,00 |
| Hydrolysat de gluten de blé | 1,00 |
| Polysorbate 20 | 1,00 |
| Carbomer (CARBOPOL ULTREZ^{®} 10) | 0,75 |
| Acétate de benzyle | 0,60 |
| Méthoxycinnamate d'octyle | 0,50 |
| Pidolate de magnésium | 1,50 |
| Eau déminéralisée | qsp 100 |

### Exemples 7 et 8

On procède comme indiqué ci-dessus aux exemples 3 et 4 en utilisant, comme composition topique, une mousse purifiante au ghassoul.

### Exemple 9

### (a) Comprimés [correspondant à l'Exemple 10 de la demande parente]

On a préparé des comprimés monocouches, à libération progressive, dosés à 50 mg de magnésium chacun et ayant la formulation suivante (le rapport pondéral B1/B2 étant de 9,15/1).

| Constituants | Qté/cp (mg) |
|---|---|
| *Noyau :* | |
| MgCl₂.9/2H₂O | 362,50 |
| HPMC | 91,50 |
| Mono-diglycéride béhénate | 10,00 |
| Lactose anhydre | 5,50 |
| Silice colloïdale anhydre | 5,50 |

| *Pelliculage 1 :* | |
|---|---|
| Gomme laque (OPAGLOS^{®} NA715G, produit commercialisé par la Sté dite COLORCON) | 1,3 à 2,2 % * |

| *Pelliculage 2* : | |
|---|---|
| mélange HPMC/HPC 1/3 p/p (OPADRY^{®} VMS, produit commercialisé par la Sté dite COLORCON) | 1,1 à 1,6 % * |
| Jaune 20A38069 | 0,008 |

| | |
|---|---|
| Note (*) : pourcentage en poids par rapport au poids du comprimé nu. | |

### (b) Gel

On fait appel au gel de l'exemple 1b.

### (c) Conditionnement et posologie

On conditionne la préparation orale (a), sous forme de comprimé retard, et la préparation topique (b) dans une même trousse, la posologie quotidienne étant de 2 comprimés contenant chacun 50 mg de Mg (une prise le matin et une prise le soir) et deux applications du gel par jour (une le matin et l'autre le soir).

### Exemple 10

### (a) Comprimés [correspondant à l'Exemple 11 de la demande parente]

On a préparé des comprimés monocouches (dosés à 50 mg en magnésium) à libération progressive et ayant la formulation suivante (le rapport pondéral B1/B2 étant de 9,15/1).

| Constituants | Qté/cp (mg) |
|---|---|
| *Noyau* : | |
| MgCl₂.9/2H₂O | 362,50 |
| HPMC | 91,50 |
| Mono-diglycéride béhénate | 10,00 |
| Lactose anhydre | 5,50 |
| Silice colloïdale anhydre | 5,50 |

| *Pelliculage* : | |
|---|---|
| Gomme laque (OPAGLOS^{®} NA715G, produit commercialisé par la Sté dite COLORCON) | 1,7 % * |

| | |
|---|---|
| Note (*) : pourcentage en poids par rapport au poids du comprimé nu. | |

### (b) Gel

On fait appel au gel de l'exemple 1b.

### (c) Conditionnement et posologie

On conditionne la préparation orale (a), sous forme de comprimé retard, et la préparation topique (b) dans une même trousse, la posologie quotidienne étant de 2 comprimés contenant chacun 50 mg de Mg (une prise le matin et une prise le soir) et deux applications du gel par jour (une le matin et l'autre le soir).

### Exemple 11

### (a) Comprimés [correspondant à l'Exemple 12 de la demande parente]

On a préparé des comprimés ayant la formulation suivante (le rapport pondéral B1/B2 étant de 9,15/1).

| Constituants | Qté/cp (mg) |
|---|---|
| *Noyau :* | |
| MgCl₂.9/2H₂O | 362,50 |
| HPMC | 91,50 |
| Mono-diglycéride béhénate | 10,00 |
| Lactose anhydre | 5,50 |
| Silice colloïdale anhydre | 5,50 |

| *Pelliculage 1* : | |
|---|---|
| Gomme laque (OPAGLOS^{®} NA715G, produit commercialisé par la Sté dite COLORCON) | 1,7 %* |

| *Pelliculage 2* : | |
|---|---|
| mélange HPMC/HPC 1/3 p/p (OPADRY^{®} VMS, produit commercialisé par la Sté dite COLORCON) | 0,5 % * |

| | |
|---|---|
| Note (*) : pourcentage en poids par rapport au poids du comprimé nu. | |

### (b) Gel

On fait appel au gel de l'exemple 1b.

### (c) Conditionnement et posologie

On conditionne la préparation orale (a), sous forme de comprimé à libération progressive, et la préparation topique (b) dans une même trousse, la posologie quotidienne étant de 2 comprimés contenant chacun 50 mg de Mg (une prise le matin et une prise le soir) et deux applications du gel par jour (une le matin et l'autre le soir).

### Exemple 12

### (a) Comprimés

On a préparé des comprimés bicouches, à libération progressive, dosés à 100 mg de magnésium chacun et contenant dans leur couche interne (le noyau) gastrorésistante 450 mg de MgCl₂.9/2H₂O (soit approximativement 62 mg de Mg), et dans leur couche externe hydrophile à libération au niveau gastrique 275 mg de MgCl₂.9/2H₂O (soit approximativement 38 mg de Mg. Après 2h dans HCl 0,1N, on a un taux de dissolution δ de l'ordre de 38 %.

### (b) Gel

On fait appel au gel de l'exemple 1b

### (c) Conditionnement et posologie

On conditionne la préparation orale (a), sous forme de comprimé à libération progressive, et la préparation topique (b) dans une même trousse, la posologie quotidienne étant de 1 comprimé contenant 100 mg de Mg (une prise le matin) et deux applications du gel par jour (une le matin et l'autre le soir).

## Revendications

1. Système à base de magnésium, utilisable pour les soins de la peau, **caractérisé en ce qu'**il comprend :
(a) une première source de magnésium dans une préparation orale, sous forme de comprimé à libération prolongée et continue, la dite source fournissant des ions Mg²⁺ dans l'organisme, la forme comprimé présentant *in vitro,* après 2h en milieu HCl 0,1N, un taux de dissolution (δ) du magnésium, qu'elle contient, (i) supérieur ou égal à 20 % et (ii) inférieur ou égal à 60 %, en poids par rapport au poids total du Mg apporté par ladite source, et
(b) une seconde source de magnésium dans une préparation topique, ladite source fournissant des ions Mg²⁺ au niveau de la peau.

2. Système selon la revendication 1, **caractérisé en ce que** ladite préparation orale à libération prolongée et continue est
(I) un comprimé monocouche contenant la totalité de la source de magnésium, ou
(II) un comprimé bicouche comprenant
(a) une première couche gastrorésistante, ou logée dans une enveloppe gastrorésistante, ladite première couche contenant 80 à 40 % du magnésium apporté par la source de magnésium, et
(b) une seconde couche, qui est hydrophile, entoure ladite première couche, se dissout au niveau de l'estomac et contient 20 à 60 % du magnésium apporté par la source de magnésium.

3. Système selon la revendication 2, **caractérisé en ce que** le comprimé monocouche à libération prolongée et continue, comprend une matrice pourvue d'un enrobage non gastrorésistant de protection ralentissant ou freinant la dissolution du Mg au niveau gastrique, ladite matrice étant constituée de ladite source de magnésium (A), d'un agent de retard hydrophile (B1), d'un agent de retard hydrophobe (B2), d'une charge inerte (C1) intervenant en tant que diluant et d'une charge inerte (C2) intervenant en tant que moyen lubrifiant.

4. Système selon la revendication 3 **caractérisé en ce que** ledit comprimé comprend, pour administration orale de magnésium, avec libération prolongée et continue,
• une matrice formant un noyau comprenant en mélange :
(A) 90 à 110 parties en poids de magnésium provenant d'une source choisie parmi MgO, MgCl₂, les hydrates de formule MgCl₂.n(H₂O), où n est un nombre entier ou fractionnaire ayant pour valeur 1 à 6 et de préférence 1 à 9/2, et leurs mélanges
(B1) 180 à 190 parties en poids d'hydroxypropylméthylcellulose,
(B2) 19,8 à 22,2 parties en poids de béhénate de glycéryle,
(C1) 10 à 12 parties en poids de lactose, et
(C2) 10 à 12 parties en poids de silice colloïdale ; et,
• un enrobage de protection ralentissant la libération du magnésium au niveau gastrique, qui n'est pas gastrorésistant.

5. Système selon la revendication 4, **caractérisé en ce que** ledit enrobage comprend
(D) 15 à 75 parties et de préférence 15 à 45 parties en poids d'une substance choisie parmi la gomme laque, les éthers de cellulose (notamment HPMC et HPC) et leurs mélanges.

6. Système selon la revendication 1, **caractérisé en ce que** la source de magnésium de la préparation topique est un dérivé physiologiquement acceptable de magnésium choisi parmi l'ensemble constitué par
(α) l'oxyde de magnésium, MgO,
(β) les sels de Mg avec des acides inorganiques,
(γ) les sels de Mg avec des acides organiques,
(δ) les hydrates desdits sels inorganique et organiques, et
(ε) leurs mélanges.

7. Système selon la revendications 1, **caractérisé en ce que** la source de magnésium de la préparation orale est choisie parmi l'ensemble constitué par MgO, MgCl₂, les hydrates MgCl₂.n(H₂O) où n es un nombre entier ou fractionnaire ayant pour valeur 1 à 9/2, le magnésium marin, ou un sel de Mg avec l'acide aspartique, l'acide glutamique, l'asparagine, la lysine, l'acide pidolique ou l'acide orotique.

8. Système selon l'une quelconque des revendications 1 à 7, **caractérisé en ce que** la préparation orale présente un taux de dissolution (δ) du Mg administré, déterminé d'abord par traitement dans un milieu HCl 0,1N (900 ml ; 40 °C) de T = 0 à T = 2h, puis par traitement dans un tampon (900 ml, à 40 °C) à pH 6,8 de T = 2h à T = 8h, tel que
• à T = 2h, on a : δ ≤, 60 %, plus précisément : 20 % ≤ δ ≤ 60 %, et de préférence : 25 % ≤ δ ≤ 58 % ;
• à T = 4h, on a : δ ≤ 85 %, plus précisément : 40 % ≤ δ ≤ 85 %, et de préférence : 45 % ≤ δ ≤ 82 % ;
• à T = 6h, on a : δ ≤ 98 %, plus précisément : 60 % ≤ δ ≤ 98 %, et de préférence : 80 % ≤ δ ≤ 95 % ; et
• à T = 8h, on a : δ ≤ 100 %, plus précisément : 90 % ≤ δ ≤ 100 %, et de préférence : 95 % ≤ δ ≤ 99,9 %.

9. Utilisation du système selon l'une quelconque des revendications 1 à 8 en tant que moyen cosmétique
(i) pour l'hydratation de la peau, ou
(ii) pour traiter ou prévenir le stress de la peau.

10. Système selon l'une quelconque des revendications 1 à 8 en tant que moyen cosmétique vis-à-vis du stress, de la fatigue et des déficiences de la barrière cutanée notamment en ce qui concerne l'hydratation du ***stratum corneum***.

11. Trousse de soin, **caractérisée en ce qu'**elle contient ladite préparation orale et ladite préparation topique du système suivant l'une quelconque des revendications 1 à 8.

## Patentansprüche

1. Magnesium-basiertes System, das für die Hautpflege verwendet werden kann, **dadurch gekennzeichnet, dass** es umfasst:
(a) eine erste Magnesium-Quelle in einem oralen Präparat, in Form einer Tablette mit verzögerter und kontinuierlicher Freisetzung, wobei besagte Quelle Mg²⁺-Ionen im Organismus bereitstellt, wobei die Tablettenform *in vitro,* nach 2 h in 0,1 N HCl-Medium, eine Auflösungsrate (δ) des in der Tablettenform enthaltenen Magnesiums von (i) größer als oder gleich 20 Gew.-% und (ii) kleiner als oder gleich 60 Gew.-% bezogen auf das Gesamtgewicht des von besagter Quelle bereitgestellten Mg aufweist, und
(b) eine zweite Magnesium-Quelle in einem topischen Präparat, wobei besagte Quelle Mg²⁺-Ionen der Haut bereitstellt.

2. System gemäß Anspruch 1, **dadurch gekennzeichnet, dass** besagtes orales Präparat mit verzögerter und kontinuierlicher Freisetzung
(I) eine einschichtige Tablette ist, enthaltend die gesamte Magnesium-Quelle, oder
(II) eine zweischichtige Tablette ist, umfassend
(a) eine erste Schicht, die magensaftresistent ist oder in eine magensaftresistente Umhüllung eingebaut ist, wobei besagte erste Schicht 80 bis 40% des von der Magnesium-Quelle bereitgestellten Magnesiums enthält, und
(b) eine zweite Schicht, die hydrophil ist, besagte erste Schicht umgibt, sich im Magen auflöst und 20 bis 60% des von der Magnesium-Quelle bereitgestellten Magnesiums enthält.

3. System gemäß Anspruch 2, **dadurch gekennzeichnet, dass** die einschichtige Tablette mit verzögerter und kontinuierlicher Freisetzung eine Matrix umfasst, die mit einem nicht magensaftresistenten Schutzüberzug versehen ist, der die Auflösung des Mg im Magen verlangsamt oder hemmt, wobei besagte Matrix aus besagter Magnesium-Quelle (A), einem hydrophilen Retardierungsmittel (B1), einem hydrophoben Retardierungsmittel (B2), einer inerten Ladung (C1), die als Verdünnungsmittel dient, und einer inerten Ladung (C2), die als Gleitmittel dient, besteht.

4. System gemäß Anspruch 3, **dadurch gekennzeichnet, dass** besagte Tablette für eine orale Verabreichung von Magnesium mit verzögerter und kontinuierlicher Freisetzung umfasst
• eine Matrix, die einen Kern bildet, umfassend als Gemisch:
(A) 90 bis 110 Gewichtsteile Magnesium, die aus einer Quelle stammen, ausgewählt aus MgO, MgCl₂, Hydraten der Formel MgCl₂·n(H₂O), wo n eine ganze Zahl oder eine Bruchzahl mit einem Wert von 1 bis 6 und vorzugsweise von 1 bis 9/2 ist, und Gemische davon,
(B1) 180 bis 190 Gewichtsteile Hydroxypropylmethylcellulose,
(B2) 19,8 bis 22,2 Gewichtsteile Glycerylbehenat,
(C1) 10 bis 12 Gewichtsteile Lactose, und
(C2) 10 bis 12 Gewichtsteile kolloidales Siliciumdioxid; und
• einen Schutzüberzug, der die Freisetzung des Magnesiums im Magen verlangsamt und nicht magensaftresistent ist.

5. System gemäß Anspruch 4, **dadurch gekennzeichnet, dass** besagter Überzug umfasst (D) 15 bis 75 Gewichtsteile und vorzugsweise 15 bis 45 Gewichtsteile einer Substanz, ausgewählt aus Schellack, Celluloseethern (insbesondere HPMC und HPC) und Gemischen davon.

6. System gemäß Anspruch 1, **dadurch gekennzeichnet, dass** die Magnesium-Quelle des topischen Präparates ein physiologisch verträgliches Magnesium-Derivat ist, ausgewählt aus der Gruppe, bestehend aus
(α) Magnesiumoxid, MgO,
(β) Mg-Salzen mit anorganischen Säuren,
(γ) Mg-Salzen mit organischen Säuren,
(δ) Hydraten besagter organischer oder anorganischer Salze, und
(ε) Gemischen davon.

7. System gemäß Anspruch 1, **dadurch gekennzeichnet, dass** die Magnesium-Quelle des oralen Präparates ausgewählt ist aus der Gruppe, bestehend aus MgO, MgCl₂, Hydraten MgCl₂·n(H₂O), wo n eine ganze Zahl oder eine Bruchzahl mit einem Wert von 1 bis 9/2 ist, marinem Magnesium oder einem Mg-Salz mit Asparaginsäure, Glutaminsäure, Asparagin, Lysin, Pidolsäure oder Orotsäure.

8. System gemäß einem der Ansprüche 1 bis 7, **dadurch gekennzeichnet, dass** das orale Präparat eine Auflösungsrate (δ) des verabreichten Magnesiums aufweist, bestimmt zuerst durch Behandlung in einem 0,1 N HCl-Medium (900 ml; 40°C) von T = 0 bis T = 2 h, anschließend durch Behandlung in einem Puffer (900 ml bei 40°C) bei pH 6,8 von T = 2 h bis T = 8 h, wie
• bei T = 2 h, δ ≤ 60%, genauer 20% ≤ δ ≤ 60% und vorzugsweise 25% ≤ δ ≤ 58% ist;
• bei T = 4 h, δ ≤ 85%, genauer 40% ≤ δ ≤ 85% und vorzugsweise 45% ≤ δ ≤ 82% ist;
• bei T = 6 h, δ ≤ 98%, genauer 60% ≤ δ ≤ 98% und vorzugsweise 80% ≤ δ ≤ 95% ist; und
• bei T = 8 h, δ ≤ 100%, genauer 90% ≤ δ ≤ 100% und vorzugsweise 95% ≤ δ ≤ 99,9% ist.

9. Verwendung des Systems gemäß einem der Ansprüche 1 bis 8 als kosmetisches Mittel
(i) für die Hydratation der Haut, oder
(ii) für die Behandlung oder Prävention von Hautstress.

10. System gemäß einem der Ansprüche 1 bis 8 als kosmetisches Mittel gegen Stress, Müdigkeit und Defizite der Hautbarriere, insbesondere die Hydratation des **Stratum corneum** betreffend.

11. Pflegeset, **dadurch gekennzeichnet, dass** es besagtes orales Präparat und besagtes topisches Präparat des Systems nach einem der Ansprüche 1 bis 8 enthält.

## Claims

1. A magnesium-based system which can be used for skincare, **characterized in that** it comprises:
(a) a first magnesium source in an oral preparation, in the form of a extended- and continuous-release tablet, said source supplying Mg²⁺ ions to the body, the tablet form exhibiting *in vitro,* after 2 h in 0.1N HCl medium, a rate of dissolution (δ) of the magnesium which it comprises of (i) more than or equal to 20% and (ii) less than or equal to 60%, by weight relative to the total weight of the Mg provided by said source, and
(b) a second magnesium source in a topical preparation, said source supplying Mg²⁺ ions to the skin.

2. The system as claimed in claim 1, **characterized in that** said extended-and continuous-release oral preparation is
(I) a monolayer tablet containing the entirety of the magnesium source, or
(II) a bilayer tablet comprising
(a) a first layer which is enteric, or which is housed in an enteric shell, said first layer containing 80% to 40% of the magnesium provided by the magnesium source, and
(b) a second layer, which is hydrophilic, which surrounds said first layer, which dissolves in the stomach, and which contains 20% to 60% of the magnesium provided by the magnesium source.

3. The system as claimed in claim 2, **characterized in that** the extended-and continuous-release monolayer tablet comprises a matrix having a non enteric protective coating that slows down or retards the dissolution of Mg in the stomach, said matrix being composed of said magnesium source (A), a hydrophilic retardant (B1), a hydrophobic retardant (B2), an inert filler (C1) acting as a diluent, and an inert filler (C2) acting as a lubricant.

4. The system as claimed in claim 3, **characterized in that** said tablet comprises, for oral administration of magnesium, with extended- and continuous-release,
• a matrix forming a core comprising, in a mixture:
(A) 90 to 110 parts by weight of magnesium originating from a source selected from MgO, MgCl₂, hydrates of formula MgCl₂.n(H₂O), where n is an integral or fractional number having a value of 1 to 6 and preferably 1 to 9/2, and mixtures thereof,
(B1) 180 to 190 parts by weight of hydroxypropylmethylcellulose,
(B2) 19.8 to 22.2 parts by weight of glyceryl behenate,
(C1) 10 to 12 parts by weight of lactose, and
(C2) 10 to 12 parts by weight of colloidal silica; and
• a protective coating that slows down the release of magnesium in the stomach and that is not enteric.

5. The system as claimed in claim 4, **characterized in that** said coating comprises
(D) 15 to 75 parts and preferably 15 to 45 parts by weight of a substance selected from shellac, cellulose ethers (especially HPMC and HPC), and mixtures thereof.

6. The system as claimed in claim 1, **characterized in that** the magnesium source of the topical preparation is a physiologically acceptable derivative of magnesium selected from the group consisting of
(α) magnesium oxide, MgO,
(β) salts of Mg with inorganic acids,
(γ) salts of Mg with organic acids,
(δ) hydrates of said inorganic and organic salts, and
(ε) mixtures thereof.

7. The system as claimed in claim 1, **characterized in that** the magnesium source of the oral preparation is selected from the group consisting of MgO, MgCl₂, hydrates MgCl₂.n(H₂O) where n is an integral or fractional number having a value of 1 to 9/2, marine magnesium, or a salt of Mg with aspartic acid, glutamic acid, asparagine, lysine, pidolic acid or orotic acid.

8. The system as claimed in any of claims 1 to 7, **characterized in that** the oral preparation exhibits an administered Mg dissolution rate (δ), determined first by treatment in a 0.1N HCl medium (900 ml; 40°C) from T = 0 to T = 2 h, then by treatment in a buffer (900 ml, at 40°C) at pH 6.8 from T = 2 h to T = 8 h, such that
• at T = 2 h, δ ≤ 60%, more specifically 20% ≤ δ ≤ 60%, and preferably 25% ≤ δ ≤ 58%;
• at T = 4 h, δ ≤ 85%, more specifically 40% ≤ δ ≤ 85%, and preferably 45% ≤ δ ≤ 82%;
• at T = 6 h, δ ≤ 98%, more specifically 60% ≤ δ ≤ 98%, and preferably 80% ≤ δ ≤ 95%; and
• at T = 8 h, δ ≤ 100%, more specifically 90% ≤ δ ≤ 100%, and preferably 95% ≤ δ ≤ 99.9%.

9. A use of the system as claimed in any of claims 1 to 8 as a cosmetic means for:
(i) moisturizing the skin, or
(ii) treating or preventing stress of the skin.

10. The system as claimed in any of claims 1 to 8 as a cosmetic means with regard to stress, fatigue, and deficiencies of the cutaneous barrier, particularly in respect of the moisture content of the *stratum corneum.*

11. A care kit **characterized in that** it contains said oral preparation and said topical preparation of the system as claimed in any of claims 1 to 8.
